# EUROPEAN PATENT SPECIFICATION

(11) **EP 0 242 847 B1**
(45) Date of publication and mention of the grant of the patent: **02.06.1993**
(21) Application number: 87105832.7
(22) Date of filing: 21.04.1987
(51) Int. Cl.: C07D 251/50, C07D 211/00, G01N 33/49, G01N 33/58, G01N 21/64, G01N 33/94, G01N 33/533

(54) **Tracers for use in flecainide fluorescence polarization immunoassay**
Tracer zur Benutzung in immunologischen Fluoreszenz-Polarisationsverfahren zum Nachweis von Flecainid
Traceurs à utiliser dans les essais immunologiques de fluorescence-polarisation pour déterminer le flécainide

(30) Priority: 25.04.1986 US 856079
(43) Date of publication of application: 28.10.1987
(73) Proprietor: ABBOTT LABORATORIES, North Chicago, Illinois 60064 (US)
(72) Inventor: Heiman, Daniel Feulner, Libertyville Illinois 60048 (US)
(74) Representative: Modiano, Guido, Dr.-Ing.

(56) References cited:
- EP-A- 0 199 042
- EP-A- 0 218 010
- WO-A-87/06578
- GB-A- 2 045 760
- US-A- 3 900 481

## Description

### BACKGROUND OF THE INVENTION

### Technical Field

The present invention relates to a method and reagents for a fluorescence polarization immunoassay (FPIA) procedure for determining the amount of flecainide in fluids, especially biological fluids such as serum, plasma or urine.

More specifically, the invention relates to (1) reagents for determining the amount of flecainide in a sample, viz., tracer and buffer solutions; and (2) analytical methods for conducting the assay.

### Background Art

Flecainide acetate, or N-(2-piperidylmethyl)-2,5-bis (2,2,2-trifluoroethoxy)benzamide acetate, is a drug used to treat ventricular arrhythmias and tachycardia. Although flecainide acetate has been proven to be effective clinically, it can cause undesirable side effects if optimal dosage levels are exceeded. Therapeutic blood flecainide levels have been found to range from 0.2 to 1.0 microgram per milliliter, with 0.5 microgram per milliliter considered the mean effective concentration. Monitoring of serum flecainide levels combined with clinical data can provide the physician with useful information to aid in adjusting patient dosage, achieving optimal therapeutic effects while avoiding useless subtherapeutic or harmful toxic dosage levels.

In the past, patient serum or plasma flecainide levels have typically been measured by high performance liquid chromatography (HPLC) or gas chromatography (GC). Although these methods are reasonably specific for detecting drug levels, they are not without drawbacks. They involve extensive sample preparation, time-consuming instrument set-up and a need for highly trained personnel.

In assays for other substances, competitive binding immunoassays have provided a more satisfactory alternative. Typically, competitive binding immunoassays are used for measuring ligands in a test sample. (For purposes of this disclosure, a "ligand" is a substance of biological interest to be determined quantitatively by a competitive binding immunoassay technique.) The ligands compete with a labeled reagent, or "ligand analog," or "tracer," for a limited number of receptor binding sites on antibodies specific to the ligand and ligand analog. The concentration of ligand in the sample determines the amount of ligand analog which binds to the antibody: the amount of ligand analog that will bind is inversely proportional to the concentration of ligand in the sample, because the ligand and the ligand analog each bind to the antibody in proportion to their respective concentrations.

Fluorescence polarization provides a quantative means for measuring the amount of tracer-antibody conjugate produced in a competitive binding immunoassay. Fluorescence polarization techniques are based on the principle that a fluorescent labeled compound, when excited by plane polarized light, will emit fluorescence having a degree of polarization inversely related to its rate of rotation. Accordingly, when a tracer-antibody conjugate having a fluorescent label is excited with plane polarized light, the emitted light remains highly polarized because the fluorophore is constrained from rotating between the time that light is absorbed and emitted. In contrast, when an unbound tracer is excited by plane polarized light, its rotation is much faster than that of the corresponding tracer-antibody conjugate. As a result, the light emitted from the unbound tracer molecules is depolarized

As relevant prior art, US-A-3 900 481, which discloses flecainide derivatives, may be mentioned. Furthermore, EP-A-199 042, which is considered prior art under the terms of Article 54(3) and (4) EPC, relates to fluorescein derivatives useful as tracers for procainamide. EP-A-218 010, which is also considered prior art under the terms of Article 54(3) and (4) EPC, discloses tracers for the detection of antiarrhythmic drugs characterized in that the molecule of the drug is combined with the fluorescein moiety, but EP-A-218 010 does not disclose flecainide as one of the antiarrhythmic drugs.

Fluorescence polarization immunoassay techniques are well known in the art, to date, however, the use of such techniques for the determination of flecainide levels have not been successfully attempted. Thus, the present invention offers an advance in the art in that highly sensitive tracers, a method for making the tracers, and an assay using the tracers are provided specifically for the determination of flecainide. The assay conducted in accordance with the present invention is particularly accurate, as will be explained infra.

### SUMMARY OF THE INVENTION

The present invention is directed to an FPIA for flecainide; to tracers for use in the assay; and to methods for making such tracers.

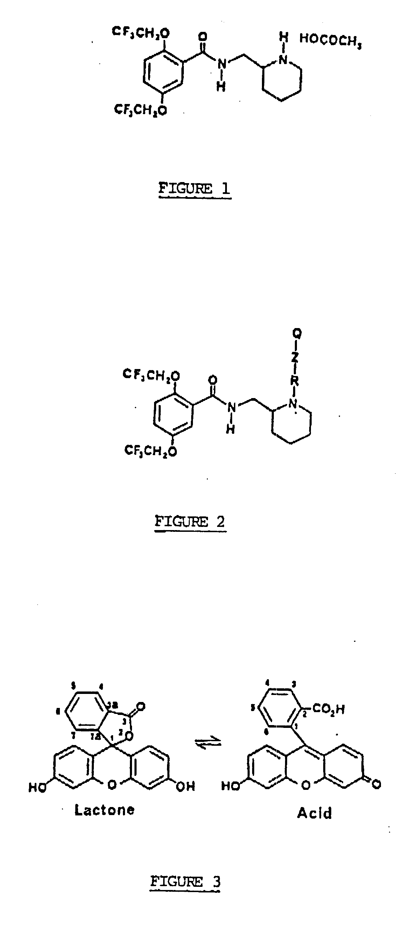

A first aspect of the invention relates to the discovery of unique tracers having novel structures. According to this first aspect of the invention, the tracers can be represented by the structural formula shown in Figure 2 of the drawings, wherein:
Q is fluoresceinyl; and
RZ is a group selected from 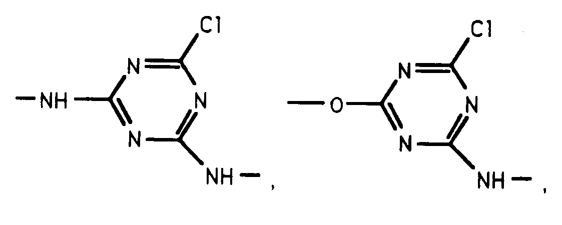
-NH-CO-, -CO-NH-, -C(NH)-NH-, -NH-CO-NH-, -NH-CS-NH-, -O-CO-NH-, -O-CS-NH-, 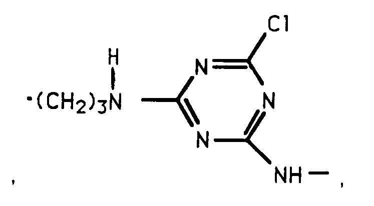
-SO₂-NH-, -O-CO-NH-SO₂-NH- 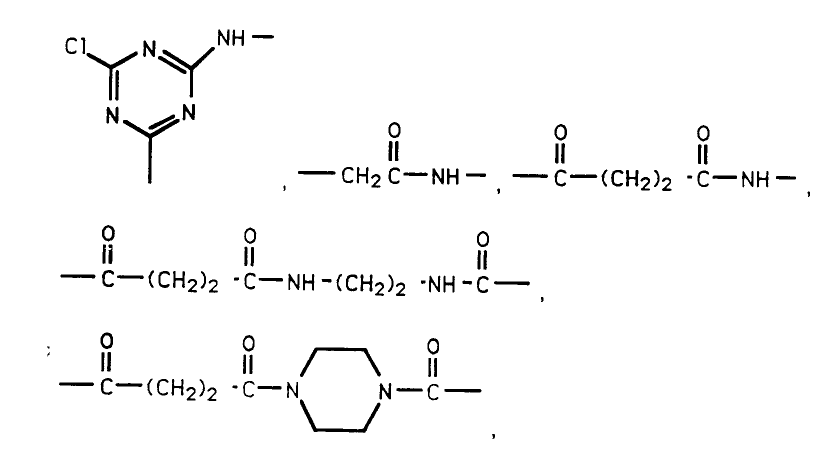 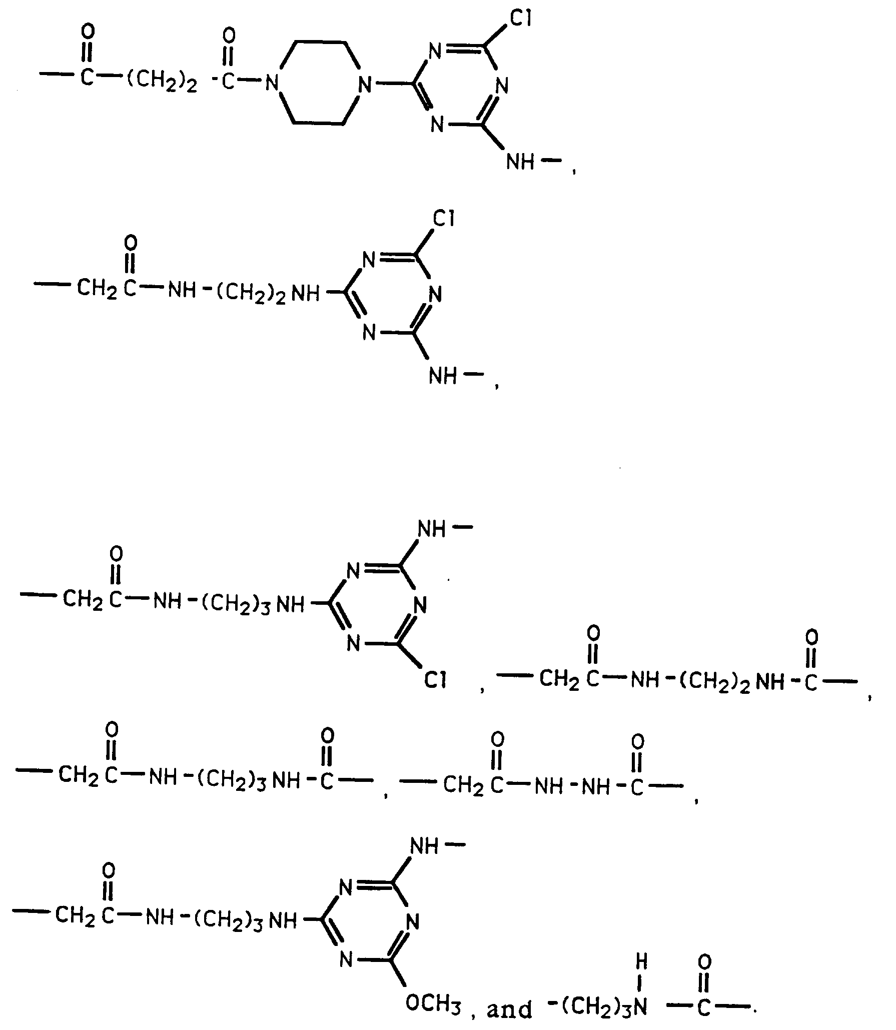
When Q is hydrogen, hydroxyl or a leaving group, the structural formula shown in Figure 2 of the drawings represents a precursor used in preparing a tracer. For purposes of this disclosure, a "leaving group" is a halogen, an acyloxy group (including a carbonate ester), a succinimidyloxy or phthalimidyloxy group, an alkoxy or phenoxy or substituted phenoxy group, an imidazolyl group, a benzotriazolyloxy group or any of the other similar activating groups well known to those skilled in the art.

According to a second aspect of the invention, a process for measuring concentration of flecainide is provided. A sample is contacted with flecainide antiserum, and a fluorescein-containing flecainide derivative capable of producing a detectable fluorescence polarization response to the presence of the flecainide antiserum. Plane-polarized light is then passed through the solution to obtain a fluorescence polarization response, and this response is detected as a measure of the amount of flecainide in the sample.

Further objects and attendant advantages of the invention will be best understood from a reading of the following detailed description taken together with the Figures and the Examples.

### BRIEF DESCRIPTION OF THE DRAWINGS

In the following Figures, the symbol "F1" represents a fluorescein moiety, and the various other symbols are noted in the Detailed Description.

Figure 1 shows the general structure of the drug flecainide acetate to be quantitatively determined in accordance with the present invention.

Figure 2 shows a general structural formula for the tracers of the present invention, as well as the classes of reactants used in preparing them.

Figure 3 shows the alternate structural formulae and names of the fluorescein moiety included in the tracers of the present invention.

Figure 4 shows various linkages that couple the fluorescein moiety to the precursor in Figure 2, when Figure 2 represents a precursor for the tracers.

Figures 5 through 24 show various examples of structures of tracers in accordance with the present invention.

### DETAILED DESCRIPTION OF THE INVENTION

The various aspects of the invention will now be discussed in detail in relation to the Figures.

### Definitions and Explanatory Material

The present invention involves the use of fluorescein and derivatives of fluorescein. In particular, a necessary property of fluorescein and its derivatives for the usefulness of the tracer compounds is the fluorescence of fluorescein. Fluorescein exists in two tautomeric forms, illustrated in Figure 3, depending on the acid concentration (pH) of the environment. In the open (acid) form, there are a number of conjugated double bonds which make that form of fluorescein (and compounds containing a fluorescein moiety) capable of absorbing blue light and emitting green fluorescence after an excited state lifetime of about four nanoseconds. When the open and closed forms coexist, the relative concentration of molecules in the open and closed forms is easily altered by adjustment of the pH level. Generally, the tracer compounds of the present invention exist in solution as biologically acceptable salts such as sodium, potassium or ammonium, allowing the compounds to exist in their open, fluorescent form when employed in the analytical methods of the present invention. The specific salt present will depend on the buffer employed to adjust the pH level. For example, in the presence of a sodium phosphate buffer, the compounds in the present invention will generally exist in the open form, as a sodium salt.

As used herein, the term "fluorescein", either as an individual compound or as a component of a larger compound, is meant to include both the open and closed forms, if they exist for a particular molecule, except in the context of fluorescence. An open form is necessary for the fluorescence to occur.

The numbering of carbon atoms of the fluorescein molecule varies, depending upon whether the open or closed form of the molecule is considered. Accordingly, the literature concerning fluorescein and its compounds is not uniform as to carbon atom numbering. In the closed form, the para-carbon to the carbonyl of the lactone on the phenyl ring is numbered 6. In the open form, the para-carbon to the carboxylic acid group on the phenyl ring is numbered 5 (see Figure 3). In this disclosure the numbering of the closed form is adopted because the raw materials used in the syntheses are most popularly numbered with that system. The carbon atom of fluorescein and its compounds which is opposite the carboxyl group is therefore numbered "6" for the purposes of the present disclosure.

A tracer in solution which is not complexed to an antibody is free to rotate in less than the time required for absorption and re-emission of fluorescent light. As a result, the re-emitted light is relatively randomly oriented so that the fluorescence polarization of a tracer not complexed to an antibody is low, approaching zero. Upon complexing with a specific antibody, the tracer-antibody complex thus formed assumes the rotation of the antibody molecule, which is slower than that of the relatively small tracer molecule, thereby increasing the polarization observed. Therefore, when a ligand competes with the tracer for antibody sites, the observed polarization of fluorescence of the resulting mixture of the free tracer and tracer-antibody complex assumes a value intermediate between that of the tracer and that of the tracer-antibody complex. If a sample contains a high concentration of the ligand, the observed polarization value is closer to that of the free ligand, i.e., low. If the test sample contains a low concentration of the ligand, the polarization value is closer to that of the bound ligand, i.e., high. By sequentially exciting the reaction mixture of an immunoassay with vertically and then horizontally polarized light and analyzing only the vertically polarized component of the emitted light, the polarization of fluorescence in the reaction mixture may be accurately determined. The precise relationship between polarization and concentration of the ligand to be determined may conveniently be established by measuring the polarization values of calibrators with known concentrations. The concentration of the ligand can be interpolated from a standard curve prepared in this manner.

The particular tracers formed in accordance with this invention have been found to produce very good assays, as discussed infra.

### The Reagents

The objective in designing a competitive binding immunoassay for flecainide is to set up a reasonably balanced competition between the drug flecainide and the tracer for the recognition sites on the antibody. Widely varying structures for haptens and tracers may be successful in achieving this goal. For the purposes of this invention, "haptens" are precursors of the immunogens, comprising generally a substituted flecainide derivative bearing a group suitable for linking to an immunologically active carrier.

### The Antibodies

The antibodies employed in the present invention are prepared by eliciting a response in rabbits or sheep to appropriate immunogens. The immunogen is administered to animals or to in vitro cultures of immunocompetent cells by a series of innoculations, in a manner well known to those skilled in the art. It should be understood that although sheep were the preferred immune host to flecainide immunogens in the experiments detailed herein, any in vivo or in vitro host capable of producing antibodies may be employed.

### The Structure of the Tracers

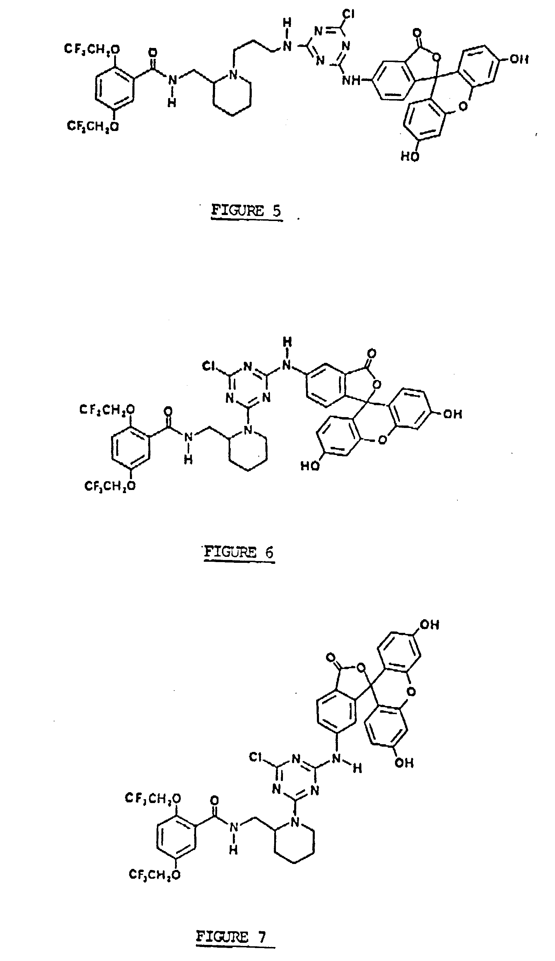

The tracers of the present invention can be represented by the general structural formula shown in Figure 2. In a preferred form of the invention, the tracer has the structural formula shown in Figure 5.

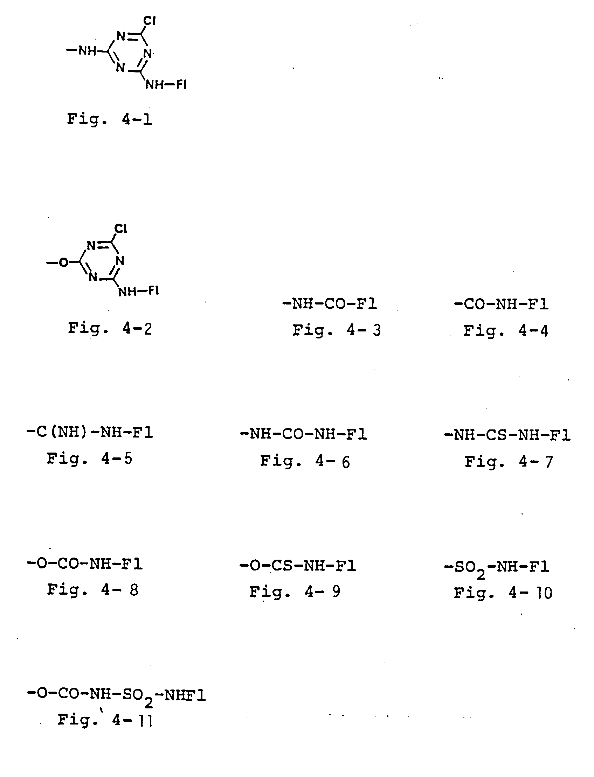

The tracer is a flecainide derivative that is linked to a fluorescein derivative by, for example, an amido, amidino, triazinylamino, carbamido, thiocarbamido, carbamoyl, sulfonamido, or sulfonylcarbamoyl group, as shown in Figure 4. The tracers are prepared by linking the appropriate fluorescein derivative to a flecainide derivative containing an amino, carboxylic acid, sulfonic acid, hydroxy, imidate, hydrazide, isocyanate, chloroformate or chlorosulfonylcarbamoyl group, as will be discussed in he context of the synthetic method and the Examples below.

By way of example, any of the following fluorescein derivatives can be used:
F1-NH₂ fluorescein amine
F1-CO₂H carboxyfluorescein
F1-NHCOCH₂I α-iodacetamidofluorescein
F1-NHCOCH₂Br α-bromoacetamidofluorescein 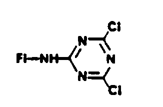
2,4-dichloro-1,3,5-triazin-2-ylaminofluorescein (DTAF) 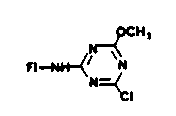
4-chloro-6-methoxy-1,3,5-triazin-2-ylaminofluorescein

### The Synthesis of the Tracers

The tracers of the present invention are made by coupling a fluorescein moiety or a derivative of fluorescein, to the general structure shown in Figure 2 wherein Q is hydrogen, hydroxyl or a leaving group.

The fluorescein moiety can be linked to the amino, carboxyl, imidate or alkoxy functional group by an amide, an amidine, a urea, a carbamate, a sulfonamide, a triazinylamino or a sulfonylcarbamate linkage, as shown in Figure 4. In the presently preferred embodiment, the fluorescein derivative is dichlorotriazinylaminofluorescein (DTAF) (Isomer I), available from Research Organics, Cleveland, Ohio, and this is coupled to a precursor shown in Figure 2 (wherein R=(CH₂)₃, Z=NH and Q=H).

The DTAF is coupled to the aminoalkyl derivative of flecainide by stirring the two compounds in methanol between a temperature of about 0°C and the boiling point of the solvent. Basic compounds such as triethylamine may be added if desired, and especially if a salt (such as the hydrochloride or acetate) of the amine precursor is employed in the reaction. Other solvents, such as dimethylformamide or dimethylsulfoxide, may be used. Usable tracers can be prepared from a variety of flecainide derivatives.

All flecainide derivatives that have a terminal amino group, such as amino, hydrazinyl or hydrazido, are coupled to carboxyfluorescein by the active ester method or the mixed anhydride method and coupled to the DTAF or alkoxy chlorotriazinylaminofluorescein by simply mixing the two materials in solution. The amino group can be converted to the isocyanate group by reaction with phosgene. This is then condensed with aminofluorescein to produce the tracer.

Flecainide derivatives having a terminal mercapto group are coupled in an inert solvent with α-bromoacetamidofluorescein or α-iodoacetamidofluorescein.

All flecainide derivatives that have a terminal carboxylic acid group, such as carboxylic acid or (aminohydroxy)alkylcarboxylic acid, are coupled to aminofluorescein by the active ester method or mixed anhydride methods.

All flecainide derivatives that have a terminal hydroxy group can be coupled to fluorescein by reaction with DTAF, α-iodoacetamidofluorescein or α-bromoacetamidofluorescein in solution. The hydroxy group can be converted to the chlorosulfonylcarbamoyl or chloroformate groups by reaction with chlorosulfonylisocyanate or phosgene, respectively. These derivatives are then coupled to aminofluorescein in solution to produce the tracer.

Sulfonic acid derivatives of flecainide are converted to the corresponding sulfonyl chlorides by reaction with a chlorinating reagent such as thionyl chloride, phosphoryl chloride or phosphorus pentachloride.

The sulfonyl halides are then reacted with aminofluoresceins or other fluorescein derivatives bearing reactive amino groups, usually in the presence of an added basic compound, to give the tracers.

Flecainide derivatives that have a terminal nitrile group are converted to imidates in anhydrous alcohol in the presence of hydrogen chloride gas. The imidate is then coupled to fluorescein amine in solution to prepare the tracer.

### The Assay

The particular tracers of the present invention have been found to measure levels of flecainide accurately and specifically in serum and plasma. Figure 1 shows the structure of the drug flecainide that can be quantitatively determined in accordance with the present invention. The assay of the present invention provides a more rapid and convenient flecainide assay method than prior art methods, because it requires no specimen treatment before analysis, the reagents are chemically and thermally stable, the assay system has minimal cross-reactivity to flecainide-like compounds and, because of its simplicity, may be carried out rapidly on highly automated equipment.

In accordance with the analytical methods of the present invention, i.e., the methods of determining flecainide by a fluorescence immunoassay procedure using the tracer compounds of the invention, a sample containing or suspected of containing flecainide is intermixed with a biologically acceptable salt of a tracer and an antibody specific to flecainide and the tracer. The flecainide and tracer compete for a limited number of antibody sites, resulting in the formation of complexes. Because the concentration of tracer and antibody is maintained constant, the ratio of flecainide antibody complex to tracer-antibody complex formed is directly proportional to the amount of flecainide in the sample. Therefore, upon exciting the mixture with linearly polarized light and measuring the polarization of the fluorescence emitted by a tracer and a tracer-antibody complex, one is able to determine quantitatively the amount of flecainide in the sample.

The results can be quantified in terms of net millipolarization units, span (in millipolarization units) and relative intensity. The measurement of millipolarization units indicates the maximum polarization when a maximum amount of the tracer is bound to the antibody in the absence of any flecainide. The higher the net millipolarization units, the better the binding of the tracer to the antibody. The span is an indication of the difference between the net millipolarization at the points of maximum and minimum amount of tracer bound to the antibody. A larger span provides for a better numerical analysis of data. The intensity is a measure of the strength of the signal above background. Thus, a higher intensity is determined at about 0.5 to 2.0 nanomolar for the preferred tracers of the invention, as the sum of the vertically polarized intensity plus twice the horizontally polarized intensity. The intensity of the tracer signal can range from about three times to about thirty times the background noise, depending upon the concentration of the tracer and other assay variables. For the purposes of the present invention, an intensity of at least five times that of background noise is preferred.

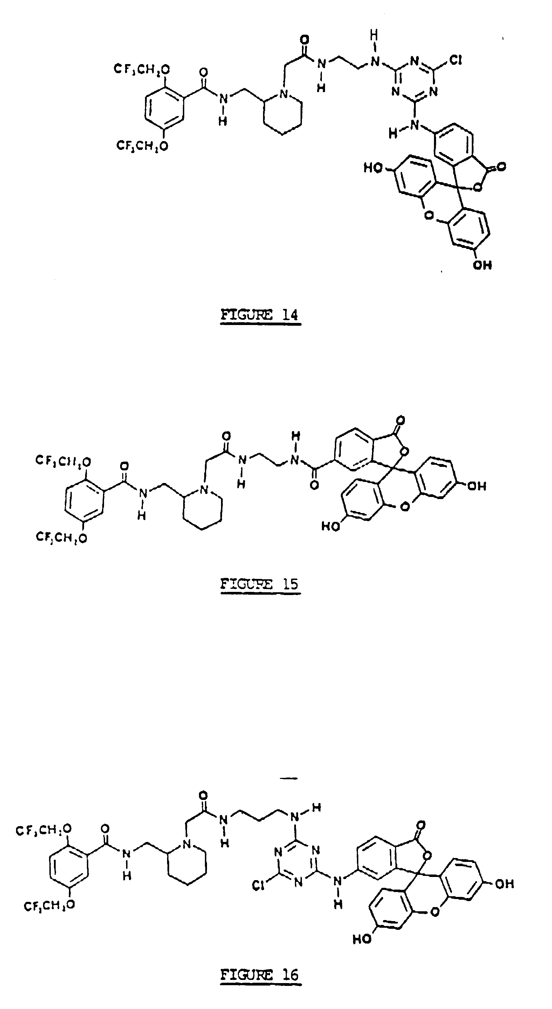

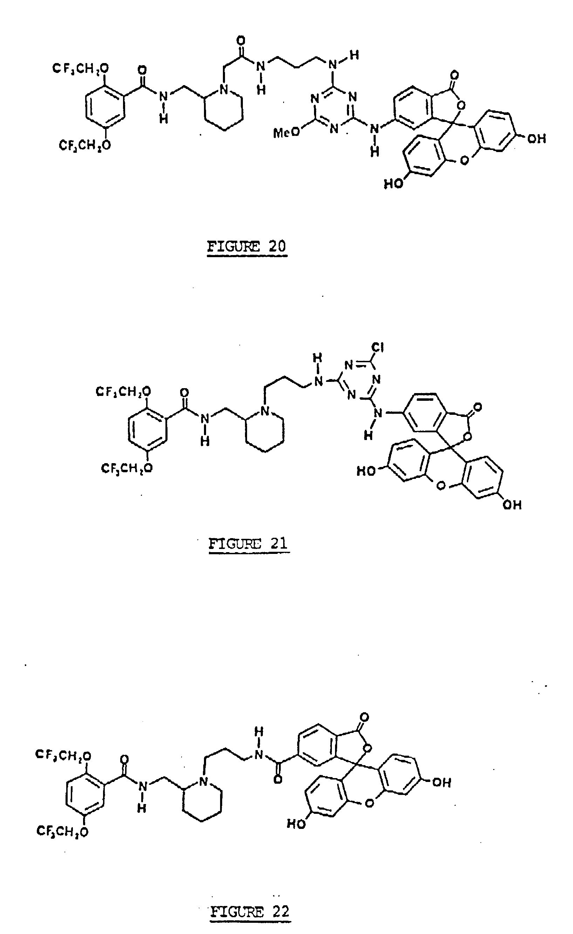

Table I shows the results obtained with various embodiments of the present invention, in terms of span, millipolarization units and intensity. In all instances, the antiserum employed was raised in sheep. As can be seen from this data, an assay produced by use of the tracer of Figure 5 provides excellent results and is presently the most preferred. In addition, the tracers represented by Figures 16 and 21 also produced acceptable results and thus are alternative preferred tracers.

**TABLE I**

| Tracer | Net Polarization* | Span* | Intensity** |
|---|---|---|---|
| Fig. 5 | 217 | 110 | 5.9 |
| Fig. 6 | 240 | 60 | 4.5 |
| Fig. 7 | 322 | 77 | 6.4 |
| Fig. 8 | 189 | 39 | 8.1 |
| Fig. 9 | 198 | 49 | 7.7 |
| Fig. 10 | 220 | 86 | 9.1 |
| Fig. 11 | 240 | 86 | 13.0 |
| Fig. 12 | 217 | 83 | 7.8 |
| Fig. 13 | 303 | 84 | 6.2 |
| Fig. 14 | 223 | 62 | 5.5 |
| Fig. 15 | 233 | 89 | 10.0 |
| Fig. 16 | 289 | 104 | 19.0 |
| Fig. 17 | 199 | 69 | 10.4 |
| Fig. 18 | 212 | 65 | 7.0 |
| Fig. 19 | 274 | 72 | 2.94 |
| Fig. 20 | 285 | 79 | 4.8 |
| Fig. 21 | 273 | 104 | 7.8 |
| Fig. 22 | 216 | 69 | 11.6 |
| Fig. 23 | 214 | 74 | 6.8 |

| | | | |
|---|---|---|---|
| * In millipolarization units | | | |
| ** Expressed as the ratio of net intensity to background noise. | | | |

The pH at which the method of the present invention is practiced must be sufficient to allow the fluorescein moiety of the tracers to exist in their open form. The pH may range from 3 to 12, more preferably in the range of from 5 to 10, and most desirably from 6 to 9. Various buffers may be used to achieve and maintain the pH during the assay procedure. Representative buffers include borate, citrate, acetate, phosphate, carbonate, tris and barbital.

The particular buffer employed is not critical to the present invention, but the tris and phosphate buffers are preferred. The cation portion of the buffer will generally determine the cation portion of the tracer salt in solution.

The preferred method of the improved assay of the present invention will now be discussed in detail. The assay is a "homogeneous assay", which means that the end polarization readings are taken from a solution in which bound tracer is not separated from unbound tracer. This represents a distinct advantage over heterogeneous immunoassay procedures wherein the bound tracer must be separated from the unbound tracer before a reading can be taken.

The reagents for the fluorescence polarization assay of the present invention comprise antibody specific for flecainide and tracer. Additionally, largely conventional solutions including a flecainide pretreatment solution, a dilution buffer, flecainide calibrators and flecainide controls are desirably prepared. Typical solutions of these reagents, some of which are described herein, are commercially available in assay "kits" from Abbott Laboratories, Abbott Park, Illinois.

All percentages expressed herein are weight/volume unless otherwise indicated. The tracer formulation presently preferred is 82 nanomolar tracer in: 0.1 molar tris buffer at pH 5.5; 5% 5-sulfosalicylate; and 0.1% sodium azide. The antiserum formulation comprises sheep antiserum diluted with: 1% normal sheep serum (volume/volume); and 0.1% sodium azide. The dilution buffer comprises: 0.1 molar sodium phosphate at pH 7.5; 0.1% sodium azide; and 0.01% bovine gamma globulin. The pretreatment formulation comprises: 0.1 molar tris buffer at pH 5.5; 5% 5-sulfosalicylate; and 0.1% sodium azide. Calibrators comprising flecainide in normal human serum at concentrations of 0.0, 0.1, 0,25, 0.50, 1.0 and 1.5 milligrams per liter, with 0.1% sodium azide preservative are useful. Controls comprising flecainide in normal human serum are provided at concentrations of 0.15, 0.60 and 1.20 milligrams per liter with 0.1% sodium azide as a preservative.

The preferred procedure is especially designed to be used in conjunction with the Abbott TDx® polarization analyzer available from Abbott Laboratories, Irving, Texas. 50 microliters of serum or plasma are required. The calibrators, controls, or unknown samples are pipetted directly into the sample well of the TDx® sample cartridge. One of the advantages of this procedure is that the sample does not require any special preparation. If a TDx® Flecainide assay kit is being used with the TDx® analyzer, samples are placed directly into a sample carousel, the caps from each of the three reagent containers in the kit are removed and placed into designated wells inside the TDx® analyzer, and the assay procedure from this point is fully automated.

If a manual assay is being performed, the sample is mixed with the pretreatment solution in dilution buffer and a background reading is taken. The tracer is then mixed with the assay. The antibody is then finally mixed into the test solution. After incubation, a fluorescence polarization reading is taken.

The fluorescence polarization value of each calibrator, control or sample is determined and may be printed on the output tape of an instrument such as the Abbott TDx® polarization analyzer. A standard curve is generated in the instrument by plotting the polarization of each calibrator versus its concentration using nonlinear regression analysis. The concentration of each control or sample is read off the stored calibration curve and printed on the output tape.

With respect to the foregoing preferred procedure, it should be noted that the tracer, antibody, pretreatment solution, calibrators and controls should be stored between 2 and 8°C, while the dilution buffer should be stored at ambient temperature. A standard curve and controls should be run every two weeks, with each calibrator and control run in duplicate. Controls should be run daily and all samples can be run in replicates if so desired.

### EXAMPLES

Examples 1 through 19 describe experiments that were performed in accordance with the concepts of the present invention.

### Example 1

### Preparation of the Tracer of Figure 6

Flecainide acetate (9.5 mg) and 2-(fluorescein-5-yl)amino-4,6-dichloro-1,3,5-triazine (8.5 mg) were dissolved in 0.15 ml of methanol and stirred at ambient temperature for 3 1/2 hours. The reaction mixture was streaked directly onto a thin-layer silica gel plate and chromatographed with chloroform/methanol to give the tracer.

### Example 2

### Preparation of the Tracer of Figure 7

Flecainide acetate (9.5 mg) and 2-(fluorescein-6-yl)amino-4,6-dichloro-1,3,5-triazine (8.0 mg) were taken up on 0.15 ml of methanol and stirred at ambient temperature for 16 hours. The reaction mixture was streaked onto a thin-layer silica gel plate and chromatographed with chloroform/methanol to give the tracer.

### Example 3

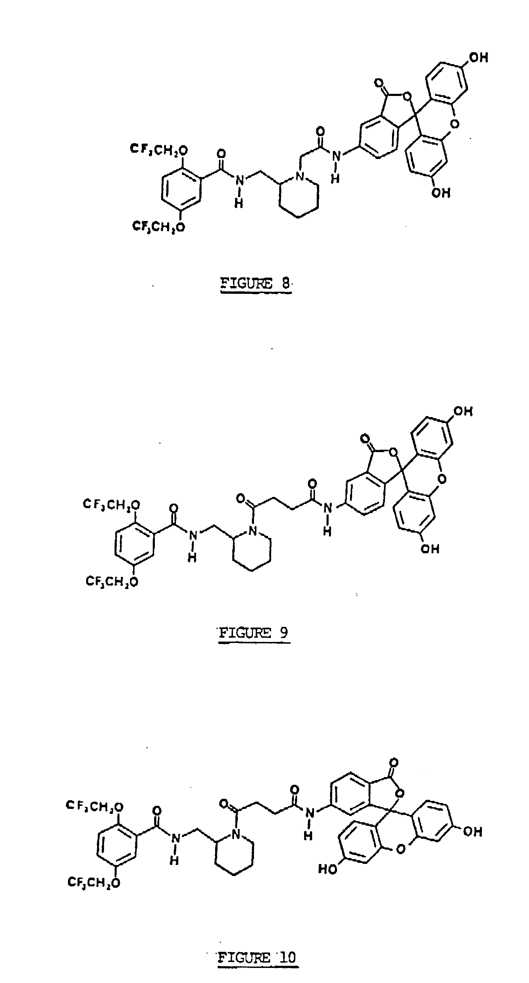

### Preparation of the Tracer of Figure 8

Flecainide acetate (23.7 mg), 5-bromoacetamidofluorescein (15.6 mg) and triethylamine (0.0116 ml) were dissolved in 0.25 ml of methanol and stirred at ambient temperature for 48 hours. The product was isolated by chromatography on a thin layer silica gel plate developed with chloroform-methanol.

### Example 4

### Preparation of the Tracer of Figure 9

Flecainide hemisuccinamide (25.7 mg), triethylamine (0.0066 ml) and isobutyl chloroformate (6.8 mg) were dissolved in 0.4 ml of dimethylformamide chilled in an ice bath and stirred for 1 hour. The solution of activated precursor was divided in two, 5-aminofluorescein (8.7 mg) was added to one portion, the mixture was allowed to warm to ambient temperature and stirring was continued for 14 hours. The solvent was removed and the residue was chromatographed on thin layer silica gel plates with chloroform-methanol and again with benzene-ethyl acetate-acetone to give the pure tracer.

### Example 5

### Preparation of the Tracer of Figure 10

One half of the solution of activated precursor prepared in Example 4 was stirred at ambient temperature with 8.7 mg of 6-aminofluorescein for 15 hours. The dimethylformamide was removed in vacuo, and the residue was chromatographed on thin layer silica gel plates with chloroform-methanol and again with benzene-ethyl acetate-acetone to give the pure tracer.

### Example 6

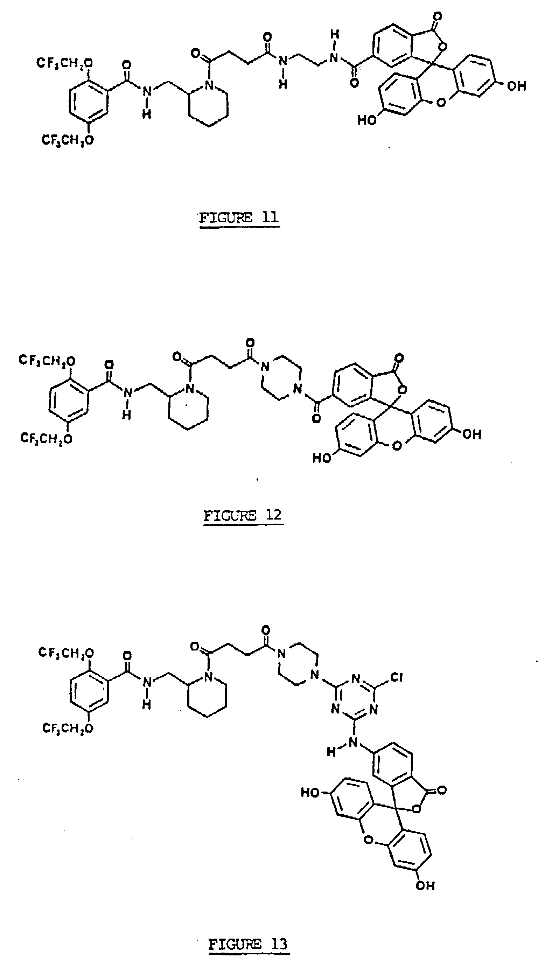

### Preparation of the Tracer of Figure 11

The flecainide-containing precursor (0.02 millimoles) and 6-(N-succinimidyloxycarbonyl)fluorescein (11.8 mg) were stirred in 0.35 ml of dimethylformamide for 4 hours at ambient temperature. The solvent was removed, and the residue was chromatographed on thin layer silica gel plates with chloroform-methanol and again with benzene-ethyl acetate-acetone to give the pure tracer.

### Example 7

### Preparation of the Tracer of Figure 12

This compound was prepared according to the method of Example 6 from 0.02 millimoles of the appropriate flecainide-containing precursor and 11.8 mg of 6-(N-succinimidyloxycarbonyl)fluorescein, chromatographing first with benzene-ethyl acetate-hexane and then with chloroform-methanol.

### Example 8

### Preparation of the Tracer of Figure 13

The flecainide-containing precursor (0.02 millimoles) and 2-(fluorescein-6-ylamino)-4,6-dichloro-1,3,5-triazine were taken up in 0.40 ml of methanol and stirred at ambient temperature overnight. The product was purified by chromatography on thin layer silica gel developed with chloroform-methanol and with benzene-ethyl acetate-acetone.

### Example 9

### Preparation of the Tracer of Figure 14

The compound was prepared according to the method of Example 8 from 0.02 millimoles of the appropriate flecainide-containing precursor and 13.3 mg of 2-(fluorescein-5-ylamino)-4,6-dichloro-1,3,5-triazine in 0.20 ml of methanol. Chromatographic purification was with chloroform-methanol.

### Example 10

### Preparation of the Tracer of Figure 15

This compound was prepared according to the method of Example 6 from 0.02 millimoles of the appropriate flecainide-containing precursor and 11.8 mg of 6-(N-succinimidyloxycarbonyl)fluorescein in 0.25 ml of dimethylformamide.

### Example 11

### Preparation of the Tracer of Figure 16

This compound was prepared according to the method of Example 8 from 0.02 millimoles of the appropriate flecainide-containing precursor and 13.3 mg of 2-(fluorescein-6-ylamino)-4,6-dichloro-1,3,5-triazine in 0.20 ml of methanol.

### Example 12

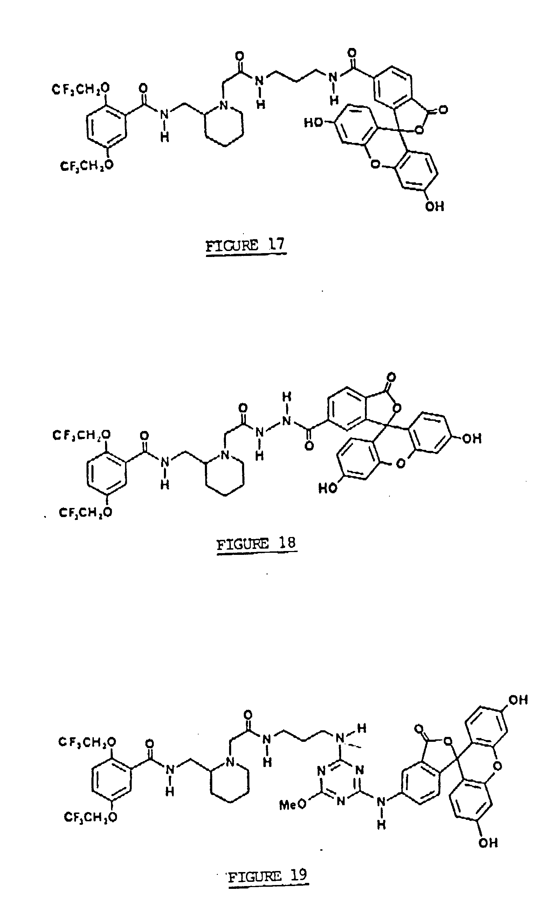

### Preparation of the Tracer of Figure 17

This compound was prepared according to the method of Example 6 from 0.02 millimoles of the appropriate flecainide-containing precursor and 11.8 mg of 6-(N-succinimidyloxycarbonyl)fluorescein in 0.25 ml of dimethylformamide. Chromatography was with chloroform-methanol.

### Example 13

### Preparation of the Tracer of Figure 18

This compound was prepared according to the method of Example 6 from 0.02 millimoles of the appropriate flecainide-containing precursor and 11.8 mg of 6-(N-succininidyloxycarbonyl)fluorescein in 0.20 ml of dimethylformamide. Chromatography was with chloroform-methanol.

### Example 14

### Preparation of the Tracer of Figure 19

A mixture of 0.02 millimoles of the flecainide-containing precursor and 13.2 mg of 2-(fluorescein-5-ylamino)-4-methoxy-6-chloro-1,3,5-triazine in 0.50 ml of methanol was stirred and heated in a 50° oil bath for 6 1/2 hours. The crude reaction mixture was streaked onto a thin-layer silica gel plate and developed with chloroform-methanol to give the pure tracer.

### Example 15

### Preparation of the Tracer of Figure 20

This compound was prepared according to the method of Example 14 from 0.02 millimoles of the flecainide-containing precursor and 13.2 mg of 2-(fluorescein-6-ylamino)-4-methoxy-6-chloro-1,3,5-triazine. Chromatography was with chloroform-methanol and benzene-ethyl acetate-acetone.

### Example 16

### Preparation of the Tracer of Figure 21

A mixture of N-(3-aminopropyl)flecainide and 5.3 mg of 2-(fluorescein-6-ylamino)-4,6-dichloro-1,3,5-triazine was stirred at ambient temperature in 0.2 ml of methanol for 24 hours. The product was purified by chromatography on silica gel thin layer plates with chloroform-methanol and benzene-ethyl acetate-acetone.

### Example 17

### Preparation of the Tracer of Figure 5

This compound was prepared according to the method of Example 16, except that the 5-isomer instead of the 6-isomer of the fluorescent labeling reagent was employed.

### Example 18

### Preparation of the Tracer of Figure 22

N-(3-aminopropyl)flecainide (4.7 mg) and 6-(N-succinimidyloxycarbonyl)fluorescein (4.7 mg) were stirred in 0.20 ml of dimethylformamide for 24 hours at ambient temperature. The solvent was removed, and the residue was chromatographed on a thin layer silica gel plate with chloroform-methanol, followed by a second chromatography on a C₁₈-reversed-phase plate developed with water-methanol containing 0.5% acetic acid.

### Example 19

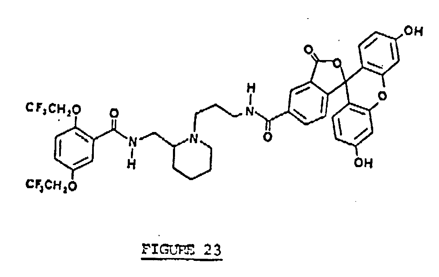

### Preparation of the Tracer of Figure 23

This compound was prepared by the method of Example 18, except that the 5-isomer of the activated carboxyfluorescein was employed instead of the 6-isomer. Purification was by chromatography twice on silica gel plates with chloroform-methanol.

### CHARACTERIZATION OF FLECAINIDE TRACERS

### Tracer of Figure 5

Ultraviolet absorbance spectrum: Lambda max= 494 nm Mass spectrum (FAB technique): (M+H)+ = 930 amu NMR spectrum (300 MHz in CD30D): delta 1.4 to 1.8 ppm, m, 8H; 2.3, m, 1H; 2.6, m, 2H; 3.05, m, 4H; 4.4 to 4.7, m, 4H; 6.6, m, 4H; 7.05 to 7.2, m, 6H; 7.35, m, 1H; 7.45, d, 1H; 7.9, t, 1H.

### Tracer of Figure 10

Ultraviolet absorbance spectrum: Lambda max = 494 nm.

### Tracer of Figure 11

Ultraviolet absorbance spectrum: Lambda max = 498.7 nm

### Tracer of Figure 15

Ultraviolet absorbance spectrum: Lambda max = 499.7 nm

### Tracer of Figure 16

Ultraviolet absorbance spectrum: Lambda max = 495.5 nm

### Tracer of Figure 21

Mass spectrum (FAB technique): (M+H)+ = 930 amu, (M+Na)+ = 952 amu, (M+2Na)+ = 974 amu.
NMR spectrum (300 MHz in CD30D: delta 1.4 ppm, m, 4H; 1.65, m, 4H, 2.05, m, 1H; 2.25, m, 2H; 2.7, m, 2H; 3.05, m, 4H; 4.4 to 4.6, m, 4H; 6.55, m, 4H;6.55,m,4H; 7.05 to 7.15, m, 6H; 7.4, d, 1H;

## Claims

1. A compound corresponding to the structure: 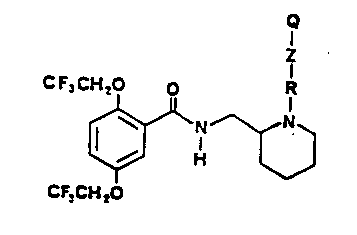 wherein
Z is NH or CO and together with R forms a group RZ selected from 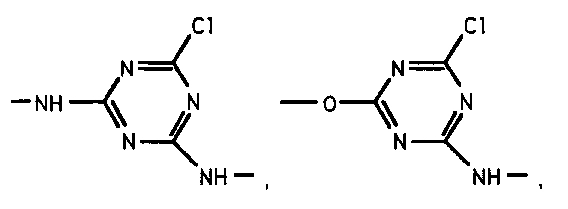 -NH-CO-, -CO-NH-, -C(NH)-NH-, -NH-CO-NH-, -NH-CS-NH-, -O-CO-NH-, -O-CS-NH-, 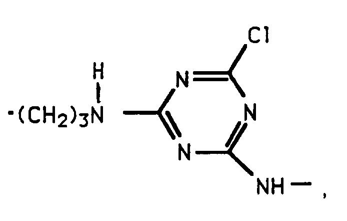 -SO₂-NH-, -O-CO-NH-SO₂-NH- 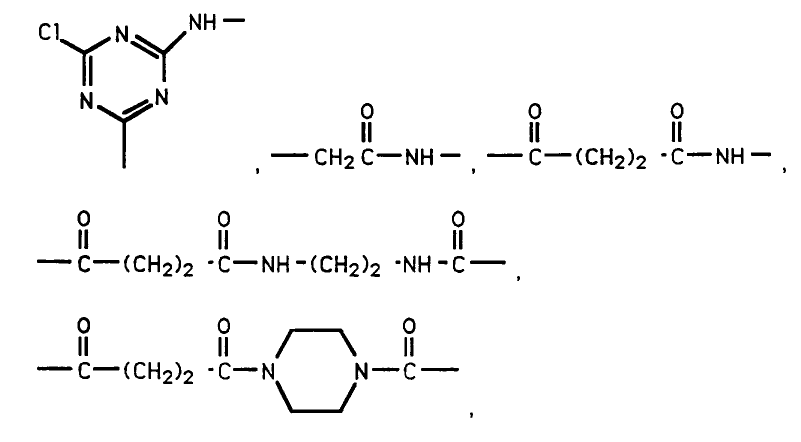 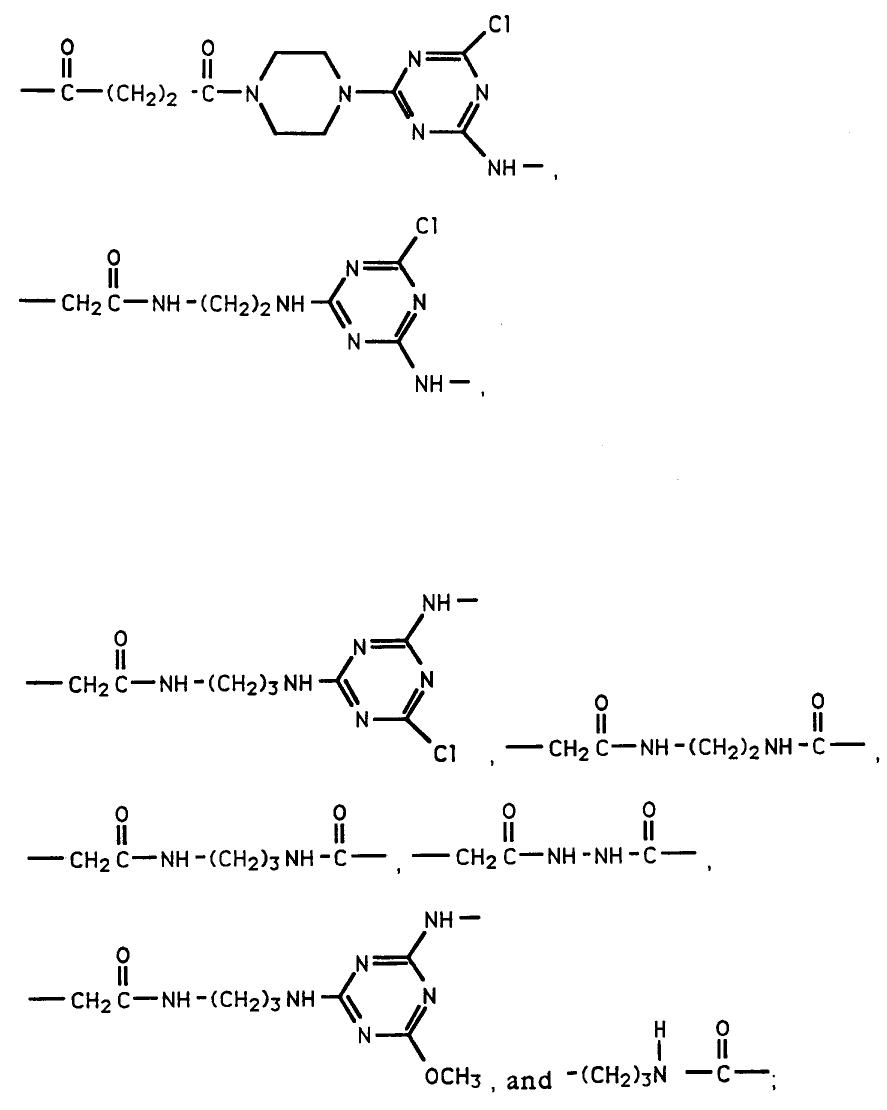 and Q is fluoresceinyl.

2. The compound of Claim 1 wherein Q-Z is (fluorescein-5-yl)amino.

3. The compound of Claim 1 wherein Q-Z is (fluorescein-6-yl)amino.

4. The compound of Claim 1 wherein Q-Z is (fluorescein-5-yl)carbonyl.

5. The compound of Claim 1 wherein Q-Z is (fluorescein-6-yl)carbonyl.

6. The compound of Claim 1 wherein -R-Z-Q has the following structure:

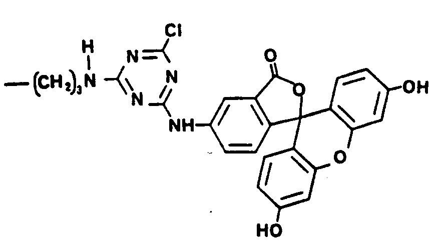

7. The compound of Claim 1 wherein -R-Z-Q has the following structure:

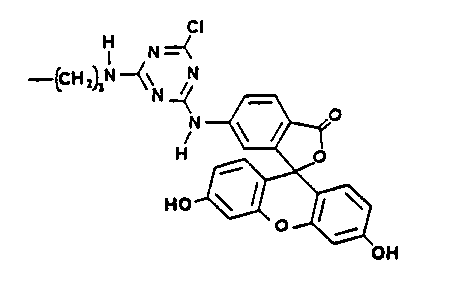

8. A process for measuring the concentration of flecainide in an aqueous solution which comprises the steps of:
a) contacting, in a buffered aqueous solution, a sample with a flecainide
antiserum and with a compound according to Claim 1 capable of producing detectable fluorescence polarization response to the presence of flecainide antiserum;
b) passing plane polarized light through the solution resulting from step a) to obtain a fluorescence polarization response; and
c) detecting the fluorescence polarization response of the solution of step b) as a measure of the amount of flecainide in the sample.

## Patentansprüche

1. Verbindung, die der folgenden Struktur entspricht: 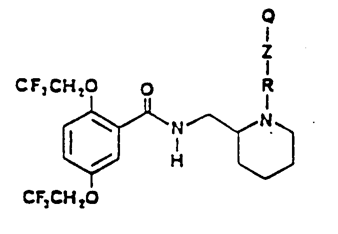 worin Z für NH oder CO steht und zusammen mit R eine Gruppe RZ bildet, die ausgewählt ist aus 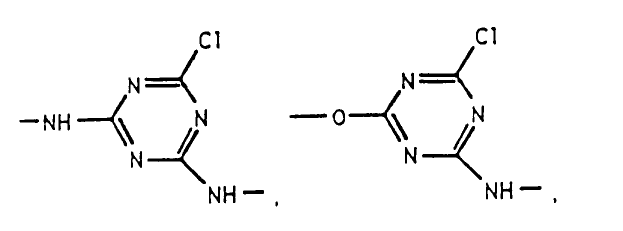 -NH-CO-, -CO-NH-, -C(NH)-NH-, -NH-CO-NH-, -NH-CS-NH-, -O-CO-NH-, -O-CS-NH-, 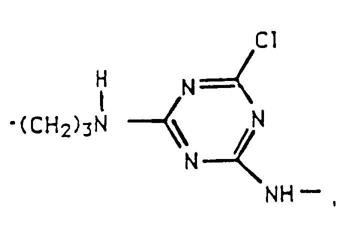 -SO₂-NH-, -O-CO-NH-SO₂-NH-, 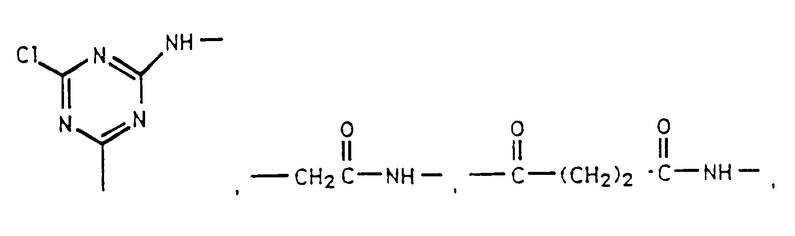 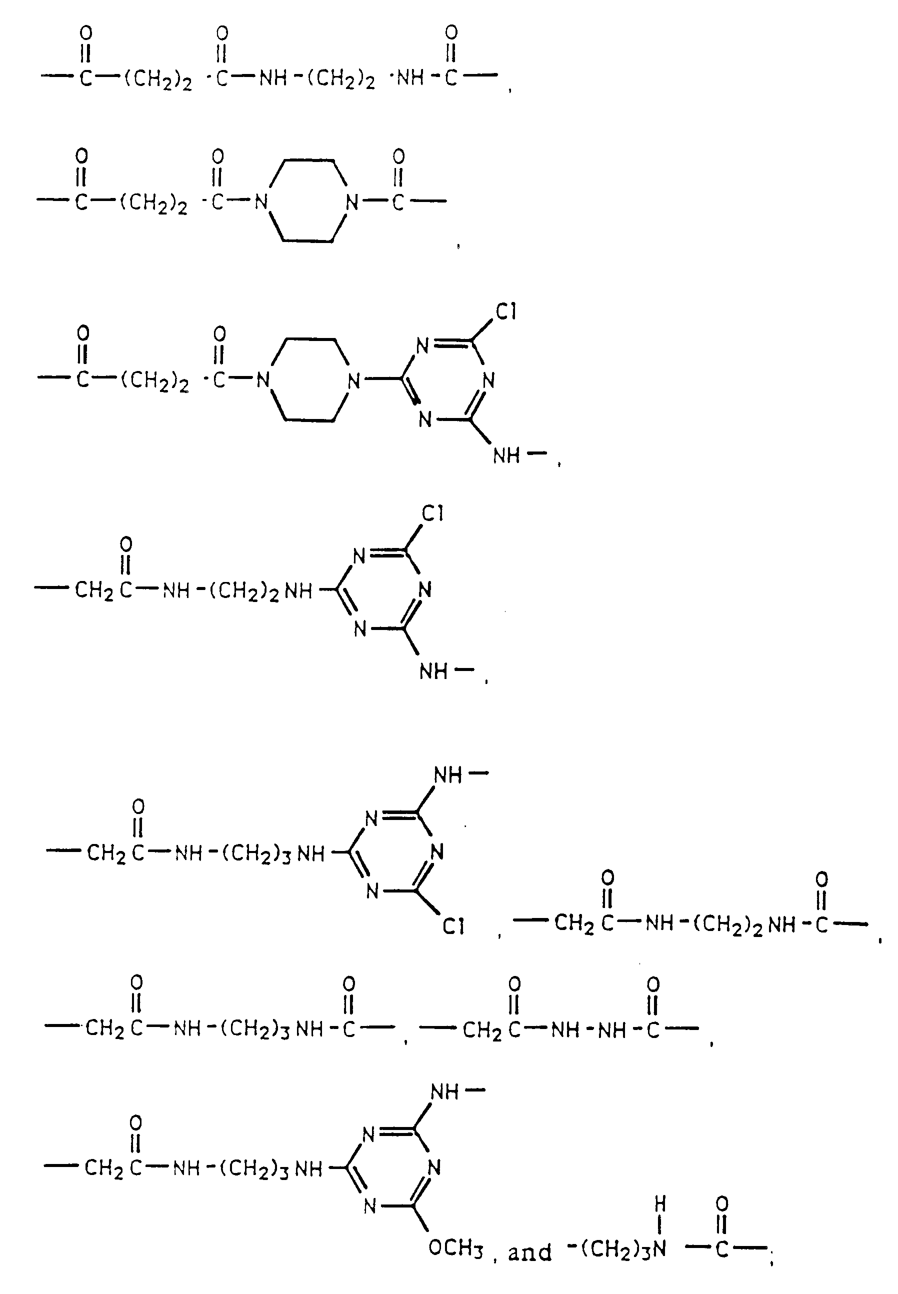 und Q Fluoresceinyl bedeutet.

2. Verbindung nach Anspruch 1, worin Q-Z (Fluorescein-5-yl)amino bedeutet.

3. Verbindung nach Anspruch 1, worin Q-Z (Fluorescein-6-yl)amino bedeutet.

4. Verbindung nach Anspruch 1, worin Q-Z (Fluorescein-5-yl)carbonyl bedeutet.

5. Verbindung nach Anspruch 1, worin Q-Z (Fluorescein-6-yl)carbonyl bedeutet.

6. Verbindung nach Anspruch 1, worin -R-Z-Q die folgende Struktur aufweist:

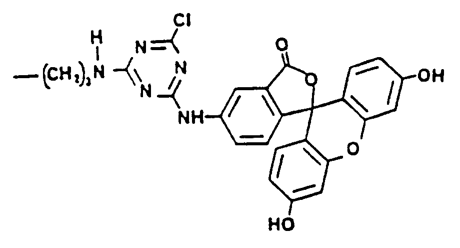

7. Verbindung nach Anspruch 1, worin -R-Z-Q die folgende Struktur aufweist:

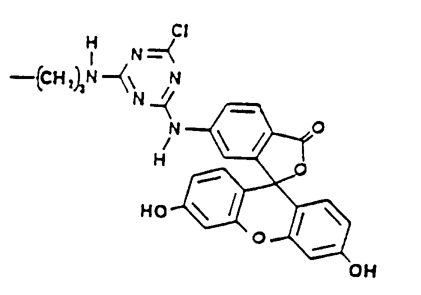

8. Verfahren zur Messung der Konzentration von Flecainid in einer wäßrigen Lösung, welches die folgenden Stufen umfaßt:
a) Kontakt in einer wäßrigen, gepufferten Lösung einer Probe mit einem Flecainid-Antiserum und einer Verbindung nach Anspruch 1, die in der Lage ist, bei Vorliegen des Flecainid-Antiserums eine detektierbare Fluoreszenz-Polarisationsantwort zu produzieren;
b) Hindurchschicken von linear polarisiertem Licht durch die in Stufe 1 erhaltene Lösung um a) eine Fluoreszenz-Polarisationsantwort zu erhalten; und
c) Detektieren der Fluoreszenz-Polarisationsantwort der Lösung aus Stufe b) als Maß für die Menge an Flecainid in der Probe.

## Revendications

1. Composition correspondant à la structure: 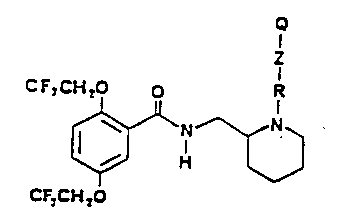 dans laquelle Z est NH ou CO et forme ensemble avec R un groupe RZ choisi parmi 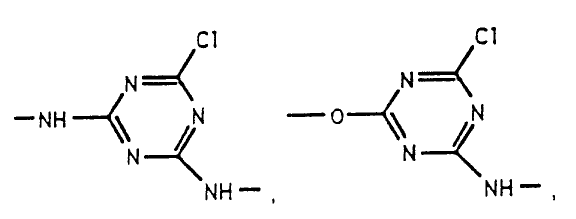 -NH-CO-, -CO-NH-, -C(NH)-NH-, -NH-CO-NH-, -NH-CS-NH-, -O-CO-NH-, -O-CS-NH-, 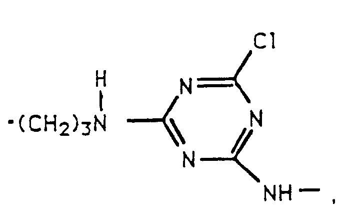 -SO₂-NH-, -O-CO-NH-SO₂-NH-, 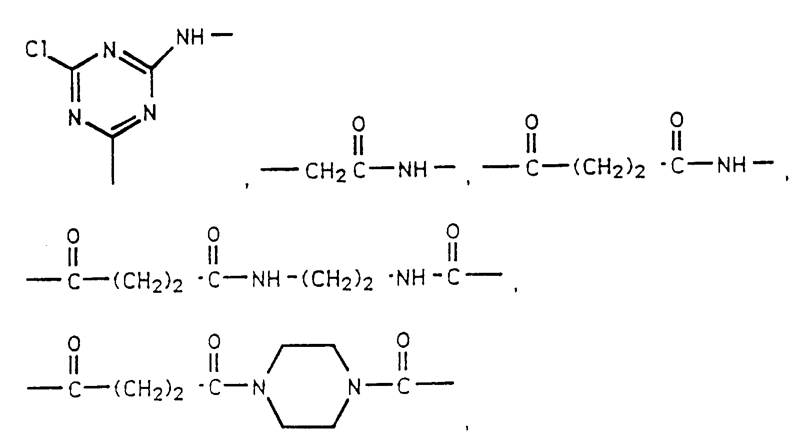 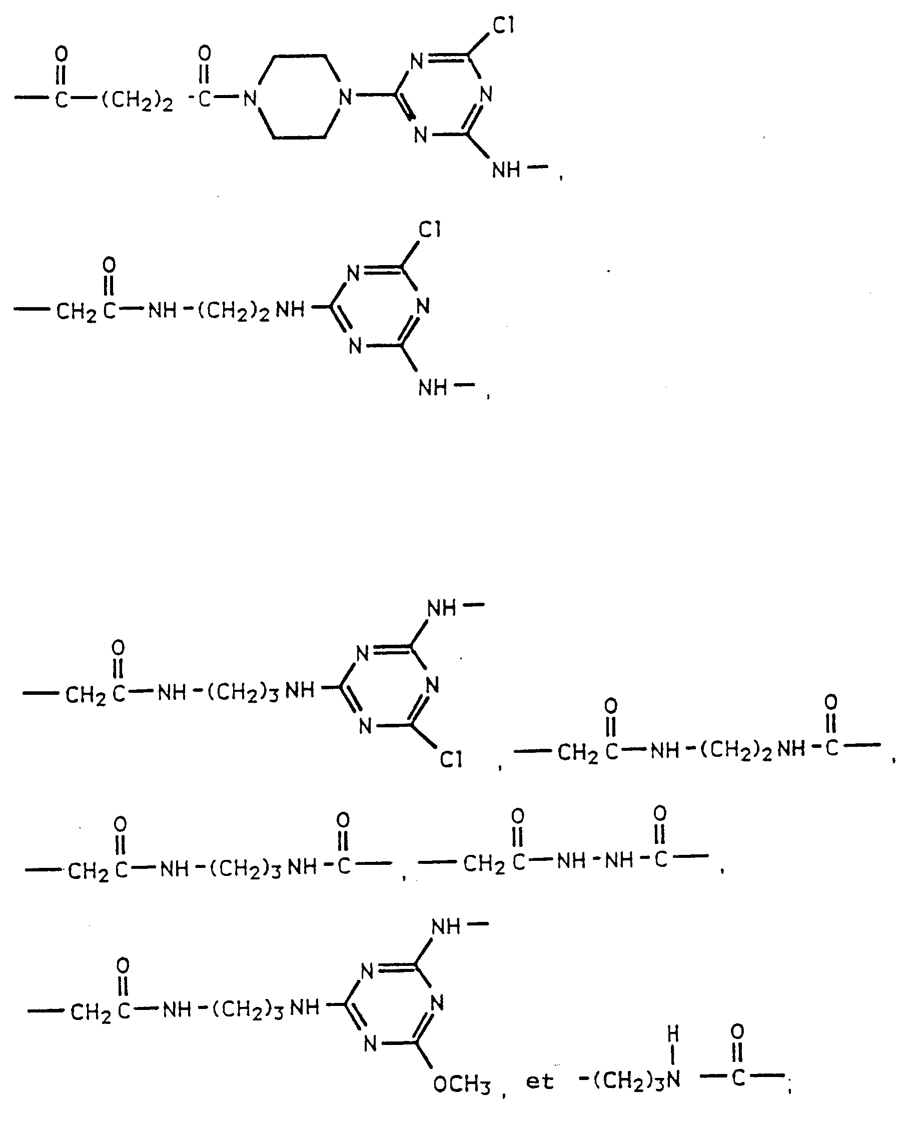 et Q est fluorescéinyle.

2. Composé selon la revendication 1, dans lequel Q-Z est (fluorescéine-5-yl)amino.

3. Composé selon la revendication 1, dans lequel Q-Z est (fluorescéine-6-yl)amino.

4. Composé selon la revendication 1, dans lequel Q-Z est (fluorescéine-5-yl)carbonyle.

5. Composé selon la revendication 1, dans lequel Q-Z est (fluorescéine-6-yl)carbonyle.

6. Composé selon la revendication 1, dans lequel -R-Z-Q a la structure suivante:

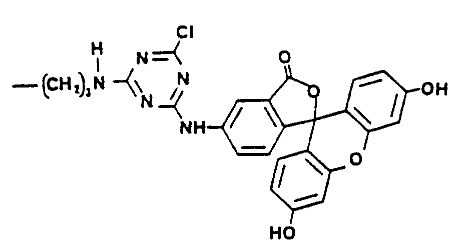

7. Composé selon la revendication 1, dans lequel -R-Z-Q a la structure suivante:

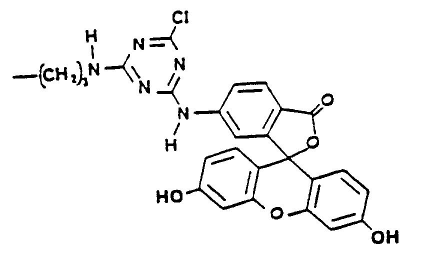

8. Procédé de mesure de la concentration de flécaïnide dans une solution aqueuse, qui comprend les étapes selon lesquelles:
a) on met en contact, dans une solution aqueuse tamponnée, un échantillon avec un antisérum de flécaïnide et avec un composé selon la revendication 1, capable de produire une réponse détectable, par polarisation de fluorescence, à la présence de l'antisérum de flécaïnide;
b) on fait passer de la lumière polarisée dans un plan à travers la solution obtenue dans l'étape a) pour obtenir une réponse par polarisation de fluorescence; et
c) on repère la réponse par polarisation de fluorescence de la solution de l'étape b) comme une mesure de la quantité de flécaïnide dans l'échantillon.
